(19) Europäisches Patentamt
European Patent Office
Office européen des brevets

(11) Veröffentlichungsnummer: **0 067 361**
**B1**

(12) # EUROPÄISCHE PATENTSCHRIFT

(45) Veröffentlichungstag der Patentschrift: **02.10.85**

(51) Int. Cl.⁴: **C 07 D 317/34**

(21) Anmeldenummer: **82104790.9**

(22) Anmeldetag: **01.06.82**

(54) **Verfahren zur Herstellung von 2-Alkoxy-(1,3)-dioxolanen.**

(30) Priorität: **11.06.81 DE 3123165**

(43) Veröffentlichungstag der Anmeldung: **22.12.82 Patentblatt 82/51**

(45) Bekanntmachung des Hinweises auf die Patenterteilung: **02.10.85 Patentblatt 85/40**

(84) Benannte Vertragsstaaten: **BE CH DE FR GB IT LI**

(56) Entgegenhaltungen:
**HOUBEN-WEYL: "Methoden der Organischen Chemie", Band VI/3, Sauerstoffverbindungen 1, Teil 3, 1965, Georg Thieme Verlag, Stuttgart, DE.**
**HOUBEN-WEYL, Methoden der Organischen Chemie Bd.1413(1965)**
**J.Polymer Sci.,Vol XXXIV (1959) S.171-179**
**K. Bodenbrenner (Dissertation) Marburg 1953**
**HOUBEN-WEYL: "Methoden der Organischen Chemie", Band VI/3, Sauerstoffverbindungen 1, Teil 3, 1965, Georg Thieme Verlag, Stuttgart, DE. VII. Cyclische Orthocarbonsäureester durch Anlagerung von 1,2-Epoxyden an Ameisensäureester oder Lactone"**

(73) Patentinhaber: **BASF Aktiengesellschaft, Carl-Bosch-Strasse 38, D-6700 Ludwigshafen (DE)**

(72) Erfinder: **Eckhardt, Heinz, Dr., Hessheimer Strasse 50, D-6710 Frankenthal (DE)**
Erfinder: **Halbritter, Klaus, Dr., Schwarzwaldstrasse 19, D-6800 Mannheim (DE)**

**EP 0 067 361 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft ein Verfahren zur Herstellung von 2-Alkoxy-(1,3)-dioxolanen durch Umsetzung von Ameisensäurealkylestern mit 2-Alkyloxiranen in Gegenwart von Borhalogeniden oder Antimon-V-halogeniden als Katalysatoren.

Es ist aus Houben-Weyl, Methoden der Organischen Chemie, Band VI/3, Seite 311 bekannt, daß man Ethylenoxid oder Epichlorhydrin mit Ameisensäureethylester in Gegenwart von Bortrifluorid zu 2-Ethoxy-1,3-dioxolanen umsetzt. Die Ausbeute im Falle von Ethylenoxid als Ausgangsstoff ist mäßig (etwa 25%). Angaben über die Verwendung höherer Epoxide fehlen.

Nach einem anderen Verfahren (Chem. Ber. 91, 650—653 (1958) kann auch 2-Ethoxy-4-methyl-1,3-dioxolan durch Umsetzung von Orthoameisensäuretriethylester und Propylenglykol in Gegenwart von Schwefelsäure erhalten werden. Die bisherigen wesentlichen Herstellungsverfahren für Orthoformiate, die für diese Umsetzung benötigt werden,

a) $HCCl_3\ 3\,NaOR \longrightarrow HC(OR)_3 + 3\,NaCl$

b) $HCN + HCl + 2\,HOR \xrightarrow[NaOR]{HCl} HC(OR)_3 + NaCl + NH_4Cl$

R = Alkylrest

zeigen Nachteile in der hohen Toxizität der Edukte ($HCCl_3$, HCN), der Umweltbelastung durch hohen Salzanfall und des Preises von Natrium.

Alle diese Verfahren befriedigen mit Bezug auf einfachen und wirtschaftlichen Betrieb sowie Ausbeute und Reinheit des Endstoffs nicht.

Es wurde nun gefunden, daß man 2-Alkoxy-(1,3)-dioxolane der Formel

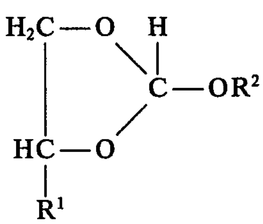

(I)

gegebenenfalls im Gemisch mit 2-Alkoxy-(1,3)-dioxolanen der Formel

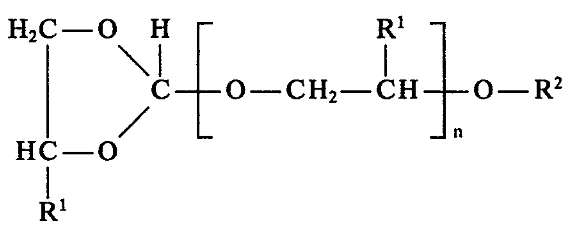

(Ia)

worin $R^1$ und $R^2$ gleich oder verschieden sind und jeweils einen Alkylrest mit 1 bis 6-Kohlenstoffatomen bedeuten und n eine ganze Zahl von 1 bis 3 bezeichnet, durch Umsetzung von Ameisensäureester mit Epoxiden in Gegenwart von Katalysatoren, vorteilhaft erhält, wenn man Ameisensäurealkylester der Formel

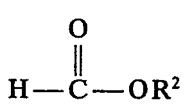

(II)

in der $R^2$ die vorgenannte Bedeutung besitzt, mit 2-Alkyloxiranen der Formel

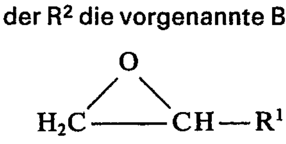

(III)

in der $R^1$ die vorgenannte Bedeutung besitzt, in Gegenwart von Borhalogeniden oder Antimon-V-halogeniden umsetzt.

Die Umsetzung kann für den Fall der Verwendung von Propylenoxid und Ameisensäureethylester durch die folgenden Formeln wiedergegeben werden:

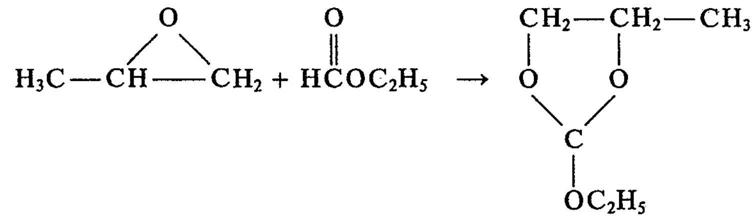

Im Vergleich zu den bekannten Verfahren liefert das Verfahren nach der Erfindung auf einfacherem und wirtschaftlicherem Wege 2-Alkoxy-(1,3)-dioxolane in besserer Ausbeute und Reinheit. Weitere Vorteile sind die geringe Toxizität der Edukte, ein sehr geringer Salzanfall (aus dem Katalysator) und nur flüssige organische Nebenprodukte. Alle diese vorteilhaften Ergebnisse des erfindungsgemäßen Verfahrens sind im Hinblick auf den Stand der Technik überraschend. Das Verfahren ist insofern unerwartet, als die kationische Polymerisation des Oxirans oder andere Nebenreaktionen durch die donatorische Wirkung der Alkylgruppe am Alkyloxiran eher begünstigt werden sollten. Tatsächlich wird aber eine geringe Bildung hochmolekularer Nebenprodukte beobachtet.

Man kann die Ausgangsstoffe II und III in stöchiometrischer Menge oder im Überschuß jeder Komponente zur anderen, zweckmäßig in einem Verhältnis von 0,001 bis 1,0, vorteilhaft von 0,03 bis 0,2 Mol Ausgangsstoff III je Mol Ausgangsstoff II umsetzen. Als Endstoff entsteht in der Regel ein Gemisch der 2-Alkoxy-(1,3)-dioxolane, zweckmäßig mit 66 bis 81 Gewichtsprozent 2-Alkoxy-(1,3)-dioxolane mit $n=0$, mit 4 bis 13 Gewichtsprozent 2-Alkoxy-(1,3)-dioxolane mit $n=1$, mit 1,0 bis 3,3 Gewichtsprozent Dioxolane mit $n=2$, mit 0,2 bis 1,0 Gewichtsprozent Dioxolane mit $n=3$, bezogen auf die Gewichtsmenge der Gesamtreaktion.

So kommen beispielsweise folgende Ausgangsstoffe II in Betracht: Ameisensäure-methyl-, -ethyl-, -propyl-, -isopropyl-, -butyl-, -isobutyl-, -sek.-butyl-, -tert.-butyl-, -pentyl-, -isopentyl-ester.

Folgende Ausgangsstoffe III sind z. B. geeignet: 2-Methyl-, 2-Ethyl-, 2-Propyl-, 2-Isopropyl-, 2-Butyl-, 2-Isobutyl-, 2-sek.-Butyl-, 2-tert.-Butyl-, 2-Pentyl-, 2-Isopentyloxiran.

Bevorzugte Katalysatoren sind Antimon-5-bromid, Antimon-V-fluorid und vorteilhaft Antimon-V-chlorid, Bortribromid, Bortrichlorid oder insbesondere Bortrifluorid. Die Konzentration des Katalysators im Reaktionsgemisch kann zwischen 0,001 und 40 Gewichtsprozent betragen, bevorzugt werden 0,05 bis 0,15 Gewichtsprozent eingesetzt. Zur Neutralisation des Katalysators, der im Reaktionsgemisch vollständig oder teilweise gelöst ist, können übliche organische oder anorganische Basen verwendet werden (z. B. Metalloxide, -carbonate, -hydroxide, -alkoholate, Amine, Amide). Zweckmäßig ist ein Verhältnis von $5 \cdot 10^{-5}$ bis 1, insbesondere von $5 \cdot 10^{-4}$ bis $15 \cdot 10^{-4}$ Mol Katalysator je Mol Ausgangsstoff II.

Die Umsetzung wird in der Regel bei einer Temperatur von $-80$ bis $+200$°C, zweckmäßig von 0 bis 100°C, vorteilhaft von 0 bis 50°C, drucklos oder unter Druck, kontinuierlich oder diskontinuierlich durchgeführt. Bevorzugt arbeitet man in einem Temperaturbereich, der zwischen 0°C und dem Siedepunkt des Reaktionsgemisches liegt. Als Lösungsmittel können z. B. aromatische und aliphatische Kohlenwasserstoffe, Halogenkohlenwasserstoffe, Carbonsäureester oder Ether verwendet werden; bevorzugt führt man die Reaktion in überschüssigem Alkylformiat durch.

Die Reaktion kann wie folgt durchgeführt werden: Ein Gemisch von Katalysator, Ausgangsstoff II und Ausgangsstoff III und gegebenenfalls von organischen Lösungsmitteln wird während 1 bis 15 Stunden bei der Reaktionstemperatur gehalten. Bei der diskontinuierlichen Verfahrensweise wird zweckmäßig der Katalysator mit Alkylformiat und Lösungsmittel vorgelegt und das Alkyloxiran oder eine Lösung des Alkyloxirans in einem Lösungsmittel, das auch Alkylformiat enthalten kann, dem Reaktionsgemisch ständig zugeführt. Nach Ende der Reaktion wird vorteilhaft der Katalysator mit Basen neutralisiert und der Endstoff in üblicher Verfahrensweise, z. B. durch Destillation, isoliert. Nach einer anderen Aufarbeitungsweise kann man nach Ende der Reaktion zunächst Lösungsmittel und überschüssiges Alkylformiat destillativ entfernen und dann den Katalysator neutralisieren.

Bei der kontinuierlichen Verfahrensweise werden vorteilhaft einem Reaktor gleichzeitig eine Lösung von $BF_3$ in einem Lösungsmittel und eine Lösung von Alkyloxiran in Alkylformiat, gegebenenfalls unter Zusatz eines weiteren Lösungsmittels, zugeführt. Das nach einer Verweilzeit von zweckmäßig 5 bis 60 Minuten aus dem Reaktor fließende Reaktionsgemisch wird in einer Destillationsapparatur von flüchtigen Bestandteilen befreit, die wieder in den Reaktor zurückgeführt werden. Die Neutralisation des Katalysators, der mit dem Reaktionsgemisch den Reaktor verläßt, kann entweder vor oder nach dieser Destillation mit einer der obengenannten Basen erfolgen. Der im fast lösungsmittelfreien Reaktionsgemisch enthaltene Endstoff wird nach üblichen Verfahrensweisen, z. B. Destillation, isoliert.

2-Alkoxy-(1,3)-dioxolane sind cyclische Orthoformiate und können wie diese als organische Zwischenprodukte verwendet werden, z. B. als $C_1$-Baustein in der Synthese von Heterocyclen, Wirkstoffen im Pharma- und Pflanzenschutzbereich, von Farbstoffen (Cyanine, Triarylmethane), als Schutzmittel bestimmter funktioneller Gruppen und als wasserbindende Mittel. Bezüglich der Verwendung wird auf die vorgenannte Literatur und Ullmanns Encyklopädie der technischen Chemie, Band 13, Seite 275, verwiesen.

Die in den folgenden Beispielen genannten Teile bedeuten Gewichtsteile.

## Beispiel 1

In einem Rührgefäß legt man 2800 Teile Methylformiat vor und versetzt mit 5 Teilen der Molekülverbindung $BF_3 \cdot HCOOCH_3$. Nach Spülen der Apparatur mit $N_2$ wird innerhalb von 6 Stunden eine Mischung von 348 Teilen Propylenoxid und 350 Teilen Methylformiat unter Rühren zugegeben; die Reaktionstemperatur steigt dabei von 22° C auf 34° C an. Man rührt noch eine Stunde nach, versetzt das Gemisch mit 10 Teilen $NaOCH_3$ und destilliert das überschüssige Methylformiat ab. Der Rückstand wird filtriert und im Vakuum fraktionierend destilliert; dabei erhält man bei 40 bis 42° C/25 mbar 530 Teile 2-Methoxy-4-methyl-(1,3)-dioxolan (75% der Theorie) und bei 69 bis 72° C/7 mbar 51 Teile 2-(2'-Methoxypropoxy)-4-methyl-(1,3)-dioxolan (10% der Theorie).

## Beispiel 2

In einem Rührgefäß legt man 1000 Teile Ethylformiat vor und versetzt mit 2 Teilen $BF_3 \cdot HCOOCH_3$. Man verfährt weiter wie in Beispiel 1, verwendet jedoch als Zulauf eine Lösung von 58 Teilen Propylenoxid in 200 Teilen Ethylformiat. Die Vakuumdestillation liefert bei 24 mbar/55 bis 70° C 109 Teile Endstoff mit einem Gehalt von 80 Gew.-% an 2-Ethoxy-4-methyl-1,3-dioxolan (66% der Theorie) und 3,5 Gew.-% 2-(2'-Ethoxypropoxy)-4-methyl-(1,3)-dioxolan (4,2% der Theorie).

## Beispiel 3

In ein Rührgefäß werden gleichzeitig zwei Lösungen von $BF_3$ und Propylenoxid in Methylformiat so zudosiert, daß die Zulaufgeschwindigkeit 1425 Teile Methylformiat/Stunde, 44 Teile Propylenoxid/Stunde und 0,7 Teile $BF_3$/Stunde betragen. Nach einer Verweilzeit von 30 Minuten wird die Reaktionslösung mit 0,7 Teilen Pyridin/Stunde versetzt und kontinuierlich bei Normaldruck destilliert. Man erhält 1315 Teile Methylformiat/Stunde (Kp 32° C) und nach Vakuumdestillation bei einer Versuchsdauer von 7,5 Stunden 267 Teile 2-Methoxy-4-methyl-(1,3)-dioxolan (63% der Theorie) und 47 Teile höhermolekulare Produkte.

## Beispiel 4

Zu 1000 Teilen einer 0,1gewichtsprozentigen Lösung von $BF_3 \cdot HCOOCH_3$ in Methylformiat werden 400 Teile einer 50gewichtsprozentigen Lösung von Propylenoxid in Methylformiat so zudosiert, daß die Zulaufgeschwindigkeit 33 Teile Propylenoxid/Stunde beträgt. Nach jeweils 60 Minuten wird eine Probe entnommen, mit $NaOCH_3$ versetzt und gaschromatographisch analysiert. Man erhält folgende Resultate:

| Zeit/h | Umsatz/% | Ausbeute/% | Selektivität/% | Verhältnis der Ausgangsstoffe III/II |
|---|---|---|---|---|
| 1 | 99,6 | 93,5 | 94,0 | 0,033 |
| 2 | 99,6 | 93,0 | 93,5 | 0,064 |
| 3 | 98,7 | 89,5 | 90,5 | 0,094 |
| 4 | 97,0 | 78,2 | 80,0 | 0,122 |
| 5 | 89,9 | 66,5 | 74,0 | 0,148 |
| 6 | 79,2 | 57,3 | 72,0 | 0,172 |
| 7 | 81,4 | 56,5 | 69,5 | 0,172 |

Beispiel 5

In einem Rührgefäß legt man 1000 Teile Methylformiat vor und versetzt mit 1 Teil der Molekülverbindung $BF_3 \cdot HCOOCH_3$. Nach Spülen der Apparatur mit $N_2$ wird innerhalb von 3,5 Stunden eine Mischung von 124 Teilen Ethyloxiran und 100 Teilen Methylformiat unter Rühren bei 32°C zugegeben. Nach Aufarbeitung wie in Beispiel 1 erhält man bei 35—125°C/25 mbar 212 Teile Endstoff mit einem Gehalt von 78,5 Gew.-% an 4-Ethyl-2-methoxy-(1,3)-dioxolan (63% der Theorie) und 7,5 Gew.-% 4-Ethyl-2-(2′-methoxybutoxy)-(1,3)-dioxolan (9% der Theorie).

Beispiel 6

In einem Rührgefäß legt man 1000 Teile Methylformiat vor und versetzt mit 5 Teilen $SbCl_5$. Nach Spülen der Apparatur mit $N_2$ wird innerhalb von 4,5 Stunden eine Mischung von 100 Teilen Propylenoxid und 100 Teilen Methylformiat bei 32°C zugegeben. Nach Aufarbeitung wie in Beispiel 1 erhält man 103 Teile 2-Methoxy-4-methyl-(1,3)-dioxolan (51% der Theorie) und 4 Teile 2-(2′-Methoxypropoxy)-4-methyl-(1,3)-dioxolan (3% der Theorie).

Beispiel 7

Man verfährt wie in Beispiel 5, jedoch mit dem Unterschied, daß als Zulauf eine Mischung von 75 Teilen Propylenoxid und 75 Teilen Methylformiat verwendet wird und man den Katalysator erst nach dem Abdestillieren des überschüssigen Methylformiats mit 1 Teil $NaOCH_3$ neutralisiert. Man erhält 132 Teile Endstoff mit einem Gehalt von 77,5 Gew.-% an 2-Methoxy-4-methyl-(1,3)-dioxolan (67% der Theorie) und 7 Gew.-% 2-(2′-Methoxypropoxy)-4-methyl-(1,3)-dioxolan (8% der Theorie).

Beispiel 8

Zu einer Lösung von einem Teil $BF_3 \cdot HCO_2CH_3$ in 500 Teilen Methylformiat wird bei 30°C innerhalb von 1,5 Stunden ein Gemisch aus 35 Teilen n-Hexyloxiran und 50 Teilen Methylformiat langsam zugegeben und das Reaktionsgemisch nach Zugabe von 2 Teilen Hexamethylendiamin zur Neutralisation des Katalysators destilliert. Man erhält 36 Teile 4-Hexyl-2-methoxy-(1,3)-dioxolan bei Kp 60—65°C/0,26 mbar (74% der Theorie).

**Patentanspruch**

Verfahren zur Herstellung von 2-Alkoxy-(1,3)-dioxolanen der Formel

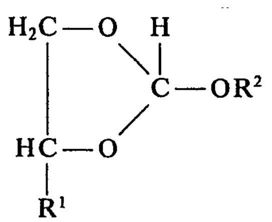

(I)

gegebenenfalls im Gemisch mit 2-Alkoxy-(1,3)-dioxolanen der Formel

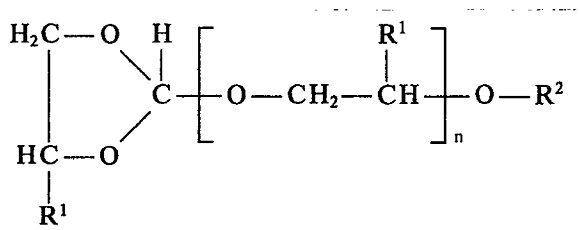

(Ia)

worin $R^1$ und $R^2$ gleich oder verschieden sind und jeweils einen Alkylrest mit 1 bis 6-Kohlenstoffatomen bedeuten und n eine ganze Zahl von 1 bis 3 bezeichnet, durch Umsetzung von Ameisensäureester

mit Epoxiden in Gegenwart von Katalysatoren, dadurch gekennzeichnet, daß man Ameisensäurealkylester der Formel

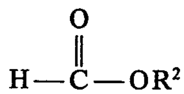

(II)

in der $R^2$ die vorgenannte Bedeutung besitzt, mit 2-Alkyloxiranen der Formel

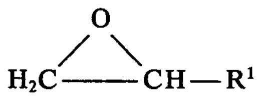

(III)

in der $R^1$ die vorgenannte Bedeutung besitzt, in Gegenwart von Borhalogeniden oder Antimon-V-halogeniden umsetzt.

## Claim

A process for the preparation of 2-alkoxy-1,3-dioxolanes of the formula

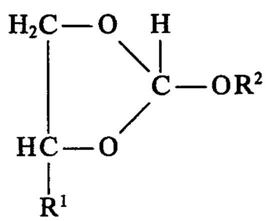

(I)

optionally in admixture with 2-alkoxy-1,3-dioxolanes of the formula

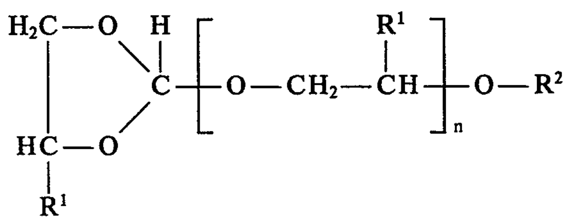

(Ia)

in which formulae $R^1$ and $R^2$ are identical or different and each is alkyl of 1 to 6 carbon atoms, and n is an integer from 1 to 3, by reacting a formate with an epoxide in the presence of a catalyst, wherein an alkyl formate of the formula

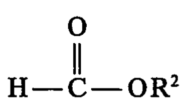

(II)

where $R^2$ has the above meaning, is reacted with a 2-alkyl-oxirane of the formula

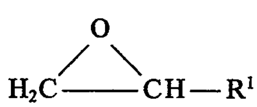

(III)

where $R^1$ has the above meaning, in the presence of a boron halide or an antimony (V) halide.

**Revendication**

Procédé de préparation de 2-alcoxy-(1,3)-dioxolanes de la formule

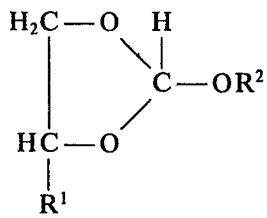

(I)

éventuellement en mélange avec des 2-alkoxy-(1,3)-dioxolanes de la formule

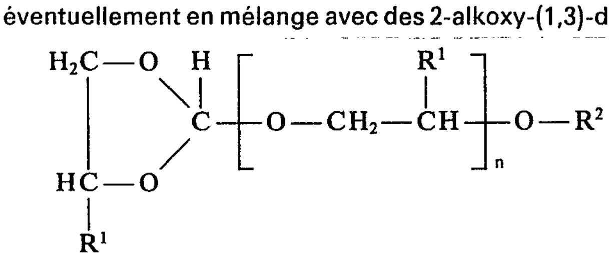

(Ia)

dans lesquelles $R^1$ et $R^2$, qui peuvent être identiques ou différents, désignent chacun un radical alkyle en $C_1$ à $C_6$ et n est un nombre entier valant de 1 à 3, par réaction d'esters de l'acide formique et d'époxydes en présence de catalyseurs, caractérisé en ce que l'on fait réagir des formiates d'alkyle de la formule

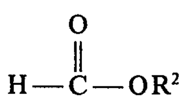

(II)

dans laquelle $R^2$ possède la signification définie, en présence d'halogénures du bore ou d'halogénures de l'antimoine pentavalent avec des 2-alkyl-oxiranes de la formule

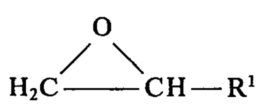

(III)

dans laquelle $R^1$ possède la signification définie.